Europäisches Patentamt

European Patent Office

Office européen des brevets

(19)

(11) Veröffentlichungsnummer: **0 074 498**
**B1**

(12) **EUROPÄISCHE PATENTSCHRIFT**

(45) Veröffentlichungstag der Patentschrift:
04.12.85

(51) Int. Cl.⁴: **A 61 B 5/00, G 01 N 27/46**

(21) Anmeldenummer: **82107376.4**

(22) Anmeldetag: **13.08.82**

(54) Verfahren und Vorrichtung zum Eichen von Messfühlern.

(30) Priorität: 04.09.81 CH 5721/81

(43) Veröffentlichungstag der Anmeldung:
23.03.83 Patentblatt 83/12

(45) Bekanntmachung des Hinweises auf die Patenterteilung:
04.12.85 Patentblatt 85/49

(84) Benannte Vertragsstaaten:
CH DE FR GB LI NL

(56) Entgegenhaltungen:
WO - A - 81/02218
GB - A - 2 030 706
GB - A - 2 054 169
US - A - 3 518 982
US - A - 3 981 297

(73) Patentinhaber: F. HOFFMANN-LA ROCHE & CO.
Aktiengesellschaft, CH-4002 Basel (CH)

(72) Erfinder: Eberhard, Patrick, Dr., Parkallee 11,
CH-4123 Allschwil (DE)
Erfinder: Mindt, Wolfgang, Dr., Sonnmattstrasse 25,
CH-4142 Münchenstein (DE)
Erfinder: Palma, Jean-Pierre, Mühleweg 17,
CH-4133 Pratteln (DE)
Erfinder: Schäfer, Robert, Dr., Peter Rot-Strasse 98,
CH-4058 Basel (DE)
Erfinder: Schärf, Robert, Grundstrasse 333,
CH-4431 Ramlinsburg (DE)

(74) Vertreter: Körber, Wolfhart, Dr. et al, Patentanwälte
Dipl.-Ing. H. Mitscherlich Dipl.-Ing. K. Gunschmann
Dr.rer.nat. W. Körber Dipl.Ing. J. Schmidt-Evers Dipl.-Ing.
W. Melzer Steinsdorfstrasse 10, D-8000 München 22 (DE)

EP 0 074 498 B1

## Beschreibung

Die Erfindung betrifft ein Verfahren zum Eichen eines Meßfühlers für die Bestimmung des Partialdrucks eines Gases, insbesondere eines kutanen Meßfühlers für die kutane Bestimmung der Sauerstoff- und der Kohlendioxyd-Konzentration im Blut, wobei der Meßfühler an ein Meßgerät angeschlossen und mit einem Eichgerät geeicht wird sowie die Eichdaten gespeichert werden. Die Erfindung betrifft ferner eine Vorrichtung zur Durchführung dieses Verfahrens mit einem Eichgerät mit einer Eichgasmischeinrichtung, einem Meßplatz, an dem der Meßfühler dem Eichgas ausgesetzt ist, und einem Meßgerät, das mit dem Meßfühler verbunden ist. Der Begriff »Eichen« soll hierbei und in der folgenden Beschreibung in einem weiteren Sinne verstanden werden und das Kalibrieren und die Funktionsprüfung, d. h. die Prüfung der Meßcharakteristik, umfassen.

Bei Sauerstofffühlern, die auf dem Prinzip der Clark-Elektrode beruhen, ist die Eichung verhältnismäßig einfach, da sie mit Umgebungsluft durchgeführt werden kann, während die Elektrode an das Meßgerät angeschlossen ist. Während der Eichung kann das Meßgerät nicht zur Patientenüberwachung eingesetzt werden. Dieses Verfahren hat also den Nachteil, daß wertvolle Betriebszeit ausfällt, und daß immer das Risiko besteht, daß ein Gerät gerade dann geeicht werden muß, wenn es dringend benötigt würde. Diesem Problem kann nur dadurch begegnet werden, daß in einer Station einer Klinik eine entsprechend größere Zahl von Geräten zur Verfügung steht.

Bei Kohlendioxydsensoren ist das Problem noch ausgeprägter. Ihre Eichung kann nicht an der Luft erfolgen, sondern muß mit einem speziellen Eichgerät vorgenommen werden, welches für die Eichung geeigneter Mischgase erzeugt. Es ist zwar im Prinzip nicht erforderlich für jedes Meßgerät ein Eichgerät vorzusehen, weil mit einem Eichgerät mehrere Meßgeräte geeicht werden können. Durch die Überschneidung der Betriebszeiten der Meßgeräte und des Eichgeräts wird aber die vorstehend für die Sauerstoff-Sensoren geschilderte Problematik noch verschärft, so daß in der Praxis oft dennoch eine größere Zahl von Eichgeräten benötigt wird, als für die Eichung einer bestimmten Anzahl von Meßgeräten eigentlich erforderlich wäre. Hinzu kommt, daß für die Eichung immer das Eichgerät zum Standort des Meßgeräts oder umgekehrt gebracht werden muß, was zu einer zusätzlichen Belastung des Klinikpersonals führt.

Der Erfindung liegt die Aufgabe zugrunde, ein Eichverfahren von Partialdruck-Meßfühlern vorzuschlagen, mit dem diese Nachteile vermieden werden können und mit dem insbesondere die Meßgeräte jederzeit und dauernd einsatzfähig sind.

Erfindungsgemäß wird dies erreicht, indem der Meßfühler mit einer Eicheinrichtung betrieben und geeicht wird, die Eichgerät und Meßgerät miteinander vereinigt; die Eichdaten in einem mit dem Meßfühler untrennbar verbundenen Speicher gespeichert werden, und bei Inbetriebnahme des Meßfühlers mit einem anderen Meßgerät die Eichdaten in das andere Meßgerät übertragen und ausgewertet werden.

Vorteilhafterweise werden mehrere Meßfühler in einer Eicheinrichtung gleichzeitig oder zyklisch hintereinander geeicht.

Ferner kann es vorteilhaft sein, gleichzeitig mit der Eichung eine Funktionskontrolle vorzunehmen.

Eine Vorrichtung zur Durchführung des erfindungsgemäßen Verfahrens zeichnet sich aus durch die bauliche Vereinigung von Meßgerät und Eichgerät in einer Eicheinrichtung; einen mit dem Meßfühler untrennbar verbundenen Datenspeicher; eine Ablaufsteuerung und eine Datenverarbeitungs- und Übertragungseinrichtung zur Einspeicherung der Eichdaten in den Datenspeicher des Meßfühlers.

Vorteilhafterweise sind mehrere Meßplätze vorhanden.

In einer besonders bevorzugten Ausführungsform der Vorrichtung ist der Datenspeicher des Meßfühlers in der Kontaktvorrichtung desselben untergebracht. Diese Kontaktvorrichtung kann beispielsweise auf galvanischer, optischer oder induktiver Kopplung beruhen.

Nachfolgend wird anhand der Zeichnungen ein Ausführungsbeispiel der Erfindung beschrieben. Es zeigt

Figur 1 eine schematische Ansicht einer Eicheinrichtung,

Figur 2 ein Blockdiagramm der wesentlichen Schaltungsteile der in Figur 1 gezeigten Einrichtung.

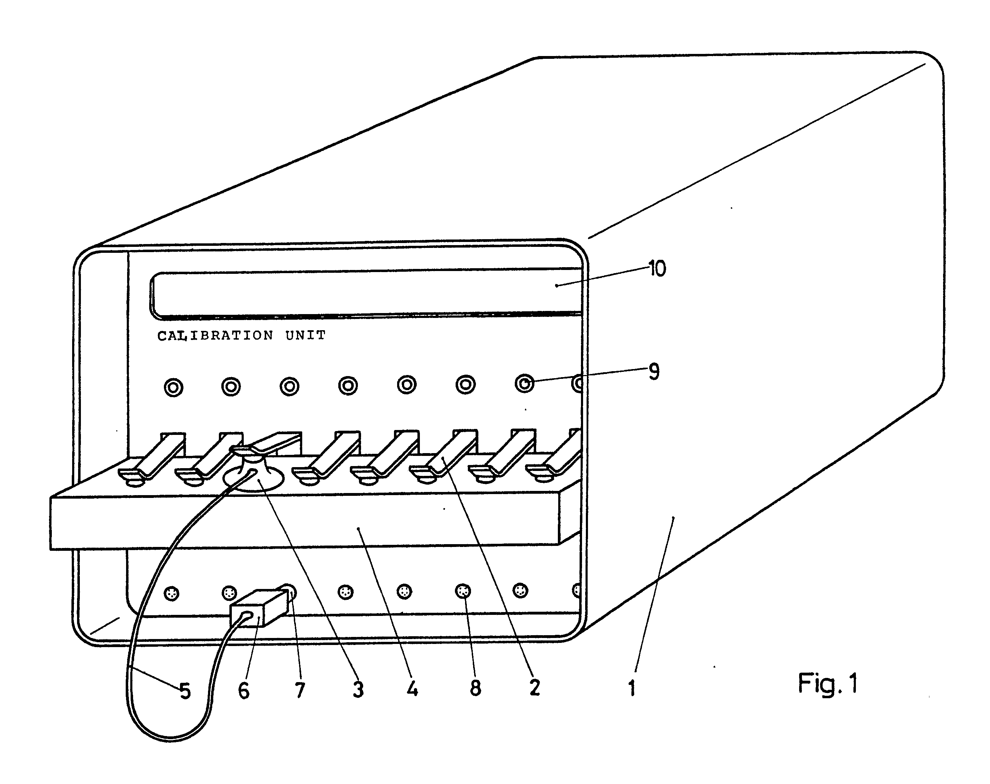

Die in Figur 1 gezeigte Eicheinrichtung 1 besitzt auf ihrer Vorderseite eine Reihe von Meßplätzen 2, die auch als Eichkammern bezeichnet werden können. Die Meßplätze 2 sind im großen und ganzen identisch mit den Meßplätzen oder Eichkammern herkömmlicher Eichgeräte wie beispielsweise dem von der Firma Kontron unter der Bezeichnung »Calibration Unit 340« vertriebenen Gerät. Im Gegensatz zu herkömmlichen Geräten weist jedoch die hier beschriebene Eicheinrichtung acht Meßplätze zur gleichzeitigen bzw. zyklisch aufeinanderfolgenden Eichung von acht Sensoren auf. Bezüglich der konstruktiven Einzelheiten der Meßplätze wird auf veröffentlichte Beschreibungen herkömmlicher Eichgeräte verwiesen.

In Figur 1 ist auf dem Meßplatz Nr. 3 eine kutane Elektrode 3 zur Eichung eingesetzt. Bei dieser Elektrode handelt es sich beispielsweise um eine herkömmliche kutane $CO_2$-Elektrode oder eine der ebenfalls bekannten kombinierten Elektroden zur Messung von $pO_2$ und $pCO_2$.

Eine nähere Beschreibung der Elektroden soll hier ebenfalls nicht vorgenommen werden. Diesbezüglich wird auf die einschlägige Literatur verwiesen.

Das die Elektrode üblicherweise mit dem Meßgerät (nicht gezeigt) verbindende Kabel 5 führt zu einer Anschlußeinheit 6, die mit einem mehrpoligen Stecker 7 versehen ist. Jedem Meßplatz 2 ist eine Buchse 8 zugeordnet, wobei in der in Figur 1 gezeigten Anordnung die Anschlußeinheit 6 mit dem Stecker 7 in die Buchse eingesteckt ist, die dem Meßplatz zugeordnet ist, auf dem sich die Elektrode 3 befindet.

Die Meßplätze 1 bis 8 sind auf einer Konsole 4 angeordnet, in der die Eichgase zu den Meßplätzen bzw. Eichkammern geführt werden. Über jedem Meßplatz befindet sich eine Anzeigelampe 9, die anzeigt, daß die Eichung und Funktionskontrolle abgeschlossen und die Elektrode zur Blutgasmessung freigegeben ist.

Oberhalb der Reihe der Anzeigelampen ist eine alphanumerische Anzeigeeinrichtung 10 angeordnet, die beim vorliegenden Ausführungsbeispiel dazu dient, bei fehlerhaften Sensoren die Art des Fehlers anzuzeigen. Darüberhinaus können selbstverständlich auch nicht-fehlerhafte Spezifikationsdaten der Sensoren angezeigt werden. Auch Angaben im Zusammenhang mit der Eichung, z. B. bei mehreren Eichgasen die Angabe des Eichgases dem der Sensor gerade ausgesetzt ist, kann vorgesehen werden.

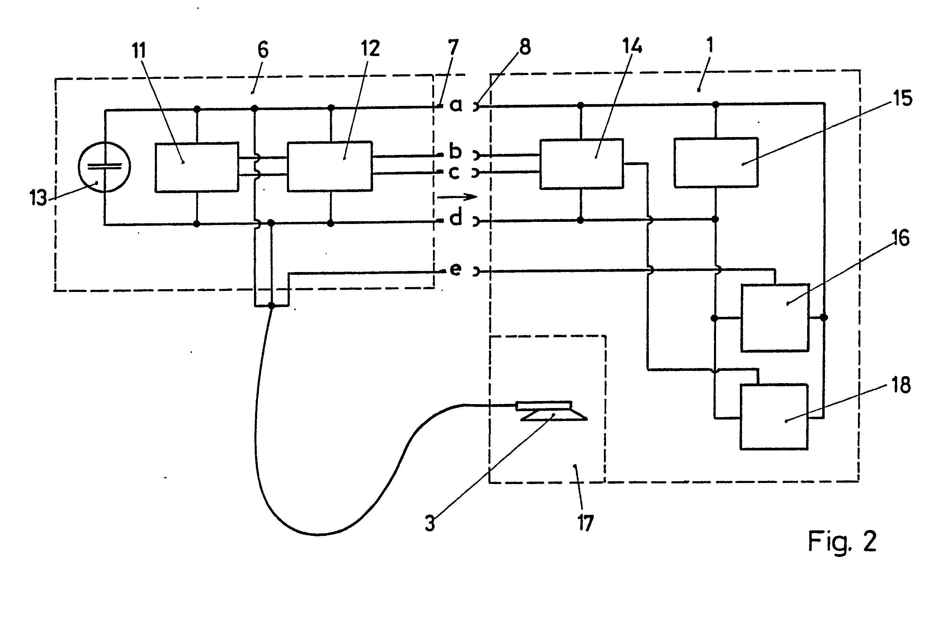

Die Anschlußeinheit 6 und die mit ihr zusammenwirkenden Teile der Eicheinrichtung sind in Figur 2 näher erläutert. Die Anschlußeinheit 6 enthält einen Speicher 11 und einen Schalter 12 zum Einschreiben in und Auslesen aus dem Speicher.

Der Speicher 11 besteht im vorliegenden Ausführungsbeispiel aus einem Schieberegister in C-MOS-Technik, wobei wahlweise die Typen 4031 (64 bit), 4517 (128 bit) oder 4537 (256 bit) eingesetzt werden können.

Anstelle des Schieberegisters kann ohne weiteres auch ein anderer Speicher vorgesehen werden, beispielsweise ein elektrisch änderbarer Schreib/Lese-Speicher (EAROM), eine Magnetkarte oder ähnliches. Auch eine Kombination verschiedener Speicher, z. B eines Schieberegisters mit einem EAROM kann je nach den gewünschten Speicherleistungen sinnvoll sein.

Der Schalter 12 dient im vorliegenden Fall, in dem das Speicherelement ein Schieberegister ist, zur Abkopplung der seriellen Dateneingabe bzw. -ausgabe. Beim vorliegenden Ausführungsbeispiel wird für den Schalter 12 ein C-MOS-Analogschalter vom Typ 4016 eingesetzt.

Die Anschlußeinheit 6 enthält neben diesen beiden Logikbausteinen auch noch einen Ladungsspeicher 13, beim vorliegenden Ausführungsbeispiel ein Kondensator mit beispielsweise 0,1 Farad Kapazität. Dieser Ladungsspeicher 13 dient zur Spannungsversorgung für das Schieberegister 11 während einer gewissen Zeitdauer, in der die Anschlußeinheit vom Gerät getrennt ist, um die gespeicherten Daten zu erhalten.

Während die Anschlußeinheit mit dem Gerät in Verbindung ist erfolgt die Spannungsversorgung über die Pole a und d des Steckers 7, über die auch der Kondensator 13 geladen wird. Die Pole b und c des Steckers 7 dienen zur Datenein- und -ausgabe. Der Pol e symbolisiert den Anschluß für die durchgehende Verbindung zu den Meßfunktionen in der Elektrode 3.

Die Verbindung der Anschlußeinheit 6 mit der Eicheinrichtung 1 erfolgt wie erwähnt über die Buchse 8. Von den Polen b und c führen Leitungen zu einer bidirektionellen Ein/Ausgabeschaltung 14, die im folgenden auch als Steuerschaltung bezeichnet wird. Diese Steuerschaltung dient zum Einschreiben in das Schieberegister, indem das Eingangssignal und das Taktsignal in zeitlich korrekter Form angelegt werden. In entsprechender Weise erfolgt auch die Datenausgabe vom Speicher 11 zum Steuergerät 14. Als Steuerschaltung wird im vorliegenden Fall ein Mikroprozessor vom Typ 8748 eingesetzt.

Zur Spannungsversorgung des Geräts, wie auch der Anschlußeinheit dient eine Speiseschaltung 15.

Der Block 16 stellt die übrige Schaltung des Meßgerätes dar, die im wesentlichen der Schaltung eines üblichen PO₂- oder PCO₂-Meßgeräts ohne die erfindungsgemäße Speicherungsmöglichkeit entspricht. Da es sich bei diesem Teil der Schaltung um herkömmliche und bekannte Ausführungsformen handelt, soll hier nicht weiter auf die Einzelheiten eingegangen werden. Es wird auf entsprechende veröffentlichte Beschreibungen verwiesen.

Eine Prozessoreinheit 18 erzeugt den für die Eichung benötigten Ablauf und übernimmt die Funktionskontrolle durch Auswertung der Daten, die vom Meßgeräteteil 16 kommen. Im vorliegenden Fall ist für diese Prozessoreinheit ein Mikroprozessor vom Typ 8085 eingesetzt.

In Figur 2 sind die Meßplätze oder Eichkammern als Teil der Eicheinrichtung in Form des gestrichelt gezeichneten Blocks 17 angedeutet. Wenn sich die Elektrode 3 wie in den Figuren 1 und 2 gezeigt auf einem Meßplatz befindet und der Stecker 7 mit der Buchse 8 verbunden ist, wie es durch den Pfeil zwischen Stecker und Buchse angedeutet ist, kann das Eichprogramm für die Elektrode 3 ablaufen, indem die Prozessoreinheit 18 und die übrige Schaltung 16 in Funktion treten. Danach werden die Eichdaten über die Steuerschaltung 14 und den Analogschalter 12 in den Speicher 11 eingegeben. Wenn dann der Sensor aus der Eicheinrichtung entnommen und mit einem Meßgerät verbunden wird, so werden die Eichdaten in das Meßgerät übertragen und bei der Messung in üblicher Weise wie die herkömmlich gemessenen Eichdaten verarbeitet. Durch den Ladungsspeicher 13 können die Eichdaten während einer gewissen Zeit gespeichert bleiben, wenn der Sensor von der Eicheinrichtung getrennt ist. Da sich die Ladung des Ladungsspeichers 13 verbraucht, verliert der Speicher 11 nach einer bestimmten Zeit seine Information. Dies ist sinnvoll, weil eine vorgenommene Eichung nicht älter als beispielsweise 24 Stunden sein sollte. Eine Eichung hat nach Ablauf eines größeren Zeitraums keine Gültigkeit mehr. Das Meßgerät enthält eine Einrichtung, die beim Einsatz eines Sensors, in dessen Speicher keine Eichdaten mehr enthalten sind, einen Fehleralarm gibt. Die entsprechende Ausführung eines solchen Warnsystems ist vom Fachmann ohne weiteres mit seinen durchschnittlichen Kenntnissen zu verwirklichen.

Die Eichdaten, d. h. die mit dem jeweiligen Sensor und vorgegebenen Eichgasen erhaltenen Meßwerte oder ein daraus ermittelter Korrekturwert werden in den Speicher 11 eingegeben. Neben diesen Eichdaten können dem Speicher auch noch weitere für den Sensor charakteristische Werte eingegeben werden. Falls der Datenspeicher in der Anschlußeinheit des Sensors auch Informationen aufnehmen soll, die nicht wie die Eichdaten von kurzer Lebensdauer sind, sondern auf Dauer gespeichert sein sollen, so muß neben dem Schieberegister ein Speicher vorgesehen sein, der die Daten unabhängig von der Spannungsversorgung durch einen Ladungsspeicher aufrecht erhält. Alternativ kann anstelle des Ladungsspeichers auch eine Batterie vorgesehen werden.

Als Daten, die typischerweise in den Speicher übertragen werden, kommen der Zeitpunkt des Eichvorganges, die Identifikation des Sensors, die Meßcharakteristik des Sensors zum Zeitpunkt der Eichung und anderes mehr in Frage.

Anstelle des Steckers 7 und der Buchsen 8 kann auch eine andere als galvanische Kopplungsart vorgesehen werden. So wäre beispielsweise eine optische oder induktive Kopplung denkbar, wodurch an dieser Stelle gleichzeitig die Erfordernisse für die Patientenisolation erfüllt würden. Wie eine solche optische oder induktive Kopplung konstruktiv gelöst wird, ist bekannt und kann vom Fachmann ohne weiteres nachvollzogen werden.

## Patentansprüche

1. Verfahren zum Eichen eines Meßfühlers (3) für die Bestimmung des Partialdrucks eines Gases, insbesondere eines kutanen Meßfühlers für die kutane Bestimmung der Sauerstoff- und Kohlendioxyd-Konzentration im Blut, wobei der Meßfühler (3) an ein Meßgerät (16) angeschlossen und mit einem Eichgerät geeicht wird sowie die Eichdaten gespeichert werden, dadurch gekennzeichnet, daß
der Meßfühler (3) mit einer Eicheinrichtung (1) betrieben und geeicht wird, die Eichgerät und Meßgerät (16) miteinander vereinigt;
die Eichdaten in einem mit dem Meßfühler (3) untrennbar verbundenen Speicher (11) gespeichert werden, und
bei Inbetriebnahme des Meßfühlers mit einem anderen Meßgerät die Eichdaten in das andere Meßgerät übertragen und ausgewertet werden.

2. Eichverfahren nach Anspruch 1, dadurch gekennzeichnet, daß mehrere Meßfühler (3) in einer Eicheinrichtung (1) gleichzeitig oder zyklisch hintereinander geeicht werden.

3. Eichverfahren nach Anspruch 1, dadurch gekennzeichnet, daß gleichzeitig mit der Eichung eine Kontrolle der Meßcharakteristik vorgenommen wird.

4. Vorrichtung zur Durchführung des Verfahrens nach einem der vorhergehenden Ansprüche mit einem Eichgerät mit einer Eichgasmischeinrichtung, einem Meßplatz (2), an dem der Meßfühler (3) dem Eichgas ausgesetzt ist, und einem Meßgerät (16), das mit dem Meßfühler (3) verbunden ist, gekennzeichnet durch
die bauliche Vereinigung von Meßgerät (16) und Eichgerät in einer Eicheinrichtung (1);
einen mit dem Meßfühler (3) untrennbar verbundenen Datenspeicher (11);
eine Ablaufsteuerung (18) und
eine Datenverarbeitungs- und Übertragungseinrichtung (12, 14) zur Einspeicherung der Eichdaten in den Datenspeicher (11) des Meßfühlers (3).

5. Vorrichtung nach Anspruch 4, dadurch gekennzeichnet, daß mehrere Meßplätze (2) vorhanden sind.

6. Vorrichtung nach Anspruch 4 oder 5, dadurch gekennzeichnet, daß der Datenspeicher (11) des Meßfühlers (3) in bzw. an einer Anschlußeinheit (6) für die Kopplung desselben mit dem Eichgerät (1) angeordnet ist.

7. Vorrichtung nach Anspruch 6, dadurch gekennzeichnet, daß die Kopplung allein oder in Kombination: galvanisch, optisch oder induktiv hergestellt ist.

## Claims

1. A method of calibrating a measuring sensor (3) for determining the partial pressure of a gas, more particularly a cutaneous measuring sensor for the cutaneous determination of the oxygen and carbon dioxide concentration of the blood, the measuring sensor (3) being connected to a meter (16) and being calibrated by a calibrator and the calibration data being stored, characterised in that:
the measuring sensor (3) is operated and calibrated with a calibrating system (1) which combines the calibrator and meter (16) together;
the calibration data are stored in a memory (11) inseparably connected to the measuring sensor (3), and
when the sensor is operated with a diferent meter the calibration data are transmitted to the different meter and evaluated.

2. A calibration method according to claim 1, characterised in that a plurality of measuring sensors (3) are calibrated simultaneously or cyclically consecutively in one calibration system (1).

3. A calibration method according to claim 1, characterised in that a check of the measuring characteristic is carried out simultaneously with the calibration.

4. Apparatus for performing the method according to any one of the preceding claims by means of a calibrator having a calibration gas mixer, a measuring station (2), at which the measuring sensor (3) is exposed to the calibration gas, and a meter (16) connected to the sensor (3), characterised by:

structural combination of meter (16) and calibrator in one calibration system (1);

a data store (11) inseparably connected to the sensor (3);

a sequence controller (18) and

a data processing and transmission system (12, 14) for entering the calibration data in the data store (11) of the measuring sensor (3).

5. Apparatus according to claim 4, characterised in that a plurality of measuring station (2) are provided.

6. Apparatus according to claim 4 or 5, characterised in that the data store (11) of the sensor (3) is disposed in or on a connecting unit (6) for coupling the same to the calibrator (1).

7. Apparatus according to claim 6, characterised in that the coupling is established by any one or more of the following in combination: electrically, optically or inductively.

**Revendications**

1. Procédé pour étalonner un capteur de mesure (3) pour la détermination de la pression partielle d'une gaz, notamment un capteur de mesure cutané pour la détermination cutanée de la teneur en oxygène et en gaz carbonique dans le sang, et selon lequel le capteur de mesure (3) est raccordé à un appareil de mesure (16) et est étalonné à l'aide d'un appareil d'étalonnage et selon lequel les données d'étalonnage sont mémorisées, caractérisé en ce que

— on fait fonctionner et on étalonne le capteur de mesure (3) avec un dispositif d'étalonnage (1), qui réunit l'un à l'autre l'appareil d'étalonnage et l'appareil de mesure (16),

— on mémorise les données d'étalonnage dans une mémoire (11) reliée d'une manière inséparable au capteur de mesure (3), et

— lors de la mise en service du capteur de mesure avec un autre appareil de mesure, les données d'étalonnage sont transférées dans l'autre appareil de mesure et sont exploitées.

2. Procédé d'étalonnage selon la revendication 1, caractérisé en ce que plusieurs capteurs de mesure (3) situés dans un dispositif d'étalonnage (1) sont étalonnés simultanément ou successivement de façon cyclique.

3. Procédé d'étalonnage selon la revendication 1, caractérisé en ce qu'un contrôle de la caractéristique de mesure est effectué en même temps que l'étalonnage.

4. Dispositif pour la mise en oeuvre du procédé selon l'une des revendications précédentes, à l'aide d'un appareil d'étalonnage comportant un dispositif de mélange d'un gaz d'étalonnage, un emplacement de mesure (2), au niveau duquel le capteur de mesure (3) est soumis au gaz d'étalonnage, et un appareil de mesure (16) qui est relié au capteur de mesure (3), caractérisé par

— la réunion, par construction, de l'appareil de mesure (16) et de l'appareil d'étalonnage dans un dispositif d'étalonnage (1),

— une mèmoire de données (11) reliée de façon inséparable au capteur de mesure (3),

— un circuit de commande d'exécution (18), et

— un dispositif (12, 14) de traitement et de transmission des données servant à mémoriser les données d'étalonnage dans la mémoire de données (11) du capteur de mesure (3).

5. Dispositif selon la revendication 4, caractérisé en ce qu'il est prévu plusieurs emplacements de mesure (2).

6. Dispositif selon la revendication 4 ou 5, caractérisé en ce que la mémoire de données (11) du capteur de mesures (3) est disposée dans ou sur une unité de raccordement (6) servant à l'accouplement de la mémoire à l'appareil d'étalonnage (1).

7. Dispositif selon la revendication 6, caractérisé en ce que l'accouplement est réalisé uniquement selon un mode galvanique, un mode optique ou un mode inductif, ou selon une combinaison de ces modes.

CALIBRATION UNIT

Fig. 1

0 074 498

Fig. 2

0 074 498